# EUROPEAN PATENT SPECIFICATION

(11) **EP 3 470 007 B1**
(45) Date of publication and mention of the grant of the patent: **28.07.2021**
(21) Application number: 17915608.8
(22) Date of filing: 30.06.2017
(51) Int. Cl.: A61C 1/08, A61C 9/00, A61B 1/247, A61C 19/04, A61B 1/00, A61B 1/045

(54) **DENTAL HANDPIECE DEVICE**
ZAHNÄRZTLICHES HANDSTÜCK
DISPOSITIF DE PIÈCE À MAIN DENTAIRE

(43) Date of publication of application: 17.04.2019
(73) Proprietor: GC Corporation, Sunto-gun, Shizuoka 410-1307 (JP)
(72) Inventor: KUME, Shunjirou, Tokyo 1748585 (JP); UENOYAMA, Takahiro, Tokyo 1748585 (JP)
(74) Representative: Müller-Boré & Partner Patentanwälte PartG mbB
(86) International application number: PCT/JP2017/024097
(87) International publication number: WO 2019/003408

(56) References cited:
- WO-A1-2004/112638
- WO-A1-2009/148044
- JP-A- H1 066 677
- JP-A- 2013 169 256
- JP-A- 2014 171 488
- US-A1- 2011 058 716
- US-A1- 2013 113 900
- US-A1- 2015 245 770

## Description

### Technical Field

The present invention relates to a dental handpiece device that photographs and displays an affected part in dental treatment.

### Background Art

In dental treatment, a dental handpiece main body is equipped with a tool, and a tooth is drilled by means of this tool. At this time, a practitioner preferably proceeds with drilling while observing a drilling state. Specifically, it is known it is important that a shape of a dental prosthesis mounted on a tooth accurately corresponds to that of a drilled tooth, which strongly influences a lifetime of a dental prosthesis. Therefore, it is very important to precisely perform drilling while observing a drilling state more clearly.

For this, Patent Literatures 1 to 5 propose a dental handpiece that has (a) built-in, or attachable and detachable camera module(s) in, or mounted on one or a plurality of places around a tool mounted part of a head part of the dental handpiece. (An) image signal(s) from the camera module(s) is/are transmitted to a video camera control unit outside the dental handpiece, and displayed on a monitor connected to the control unit as an image. Using this dental handpiece on which (a) camera module(s) is/are mounted makes it possible to observe condition of an affected part during performance of drilling on a screen of a monitor, to improve the accuracy of the operation, and the operability of the performance.

### Citation List

### Patent Literature

Patent Literature 1: JP 2015-112377 A
Patent Literature 2: JP 2014-046016 A
Patent Literature 3: JP 2013-169256 A
Patent Literature 4: JP H09-056730 A
Patent Literature 5: US 2011/0058716 A1

### Summary of Invention

### Technical Problem

However, in a conventional dental handpiece on which (a) camera module(s) is/are mounted, there is a problem of a drilled face in a blind spot according to the positional relationship of a drilling tool, (a) light entry face(s) of the camera module(s), and teeth, which makes it impossible for the camera module(s) to capture the drilled face.

In view of the above described problem, an object of the present invention is to provide a dental handpiece device that can suppress a case where a drilled face is in a blind spot and thus cannot be observed.

### Solution to Problem

The present invention solves the aforementioned problem with a dental handpiece device according to claim 1. Further advantageous aspects are defined in the dependent claims. The present invention is a dental handpiece device comprising: a dental handpiece main body; and a dental handpiece imaging device with which the dental handpiece main body is equipped, wherein the dental handpiece imaging device comprises: a plurality of camera modules each of which takes an image, to convert the image, which is taken, to an electric signal; and a processing means that receives the electric signal from each of the camera modules, to calculate for generating an image based on the electric signal, and to switch a display of the image, which is taken by each of the camera modules.

The processing means is adapted to calculate for determining whether or not a portion drilled by a tool that is mounted on the dental handpiece main body can be visually perceived.

At this time, the processing means may determine whether or not a tip of the tool can be visually perceived, to determine whether the portion can be visually perceived.

The processing means can determine whether the portion can be visually perceived using one of the camera modules which is set as a priority in advance, and when determining that the portion cannot be visually perceived, the processing means can switch the camera module of the priority to another one of the camera modules which is set as a second priority, to determine whether the portion can be visually perceived using the camera module of the second priority.

At this time, the processing means may suspend for a predetermined time before switching the camera module of the priority to the camera module of the second priority.

As another aspect of the present invention, a switch is further included, wherein the processing means may calculate so that a signal from the switch switches the image, which is taken by each of the camera modules.

### Advantageous Effects of Invention

According to the present invention, a case where a drilled face is in a blind spot and cannot be seen when a tooth is drilled can be suppressed.

### Brief Description of Drawings

Fig. 1 is an explanatory view of one embodiment, which schematically shows structure of a dental handpiece device 100.
Fig. 2 is a plan view around a head.
Fig. 3A and 3B are explanatory views of structure of a light entry and outgo means 13.
Fig. 4 is a block diagram schematically showing structure of a dental handpiece imaging device 10.
Fig. 5 is an explanatory flowchart of an example of calculations carried out by a processing means 21.

### Description of Embodiments

The present invention will be described below based on the embodiment shown in the drawings. The present invention is not restricted to this embodiment.

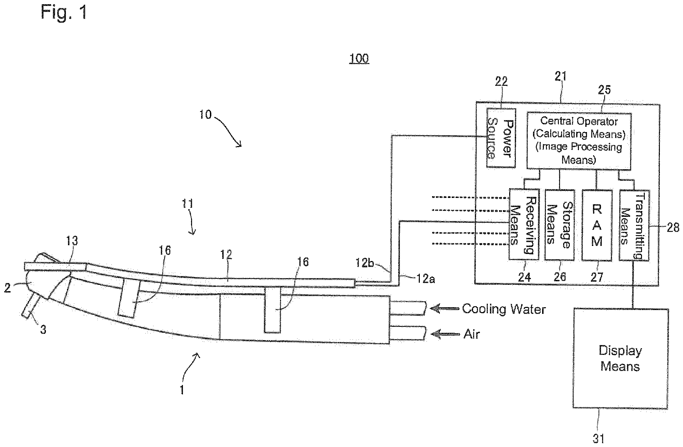

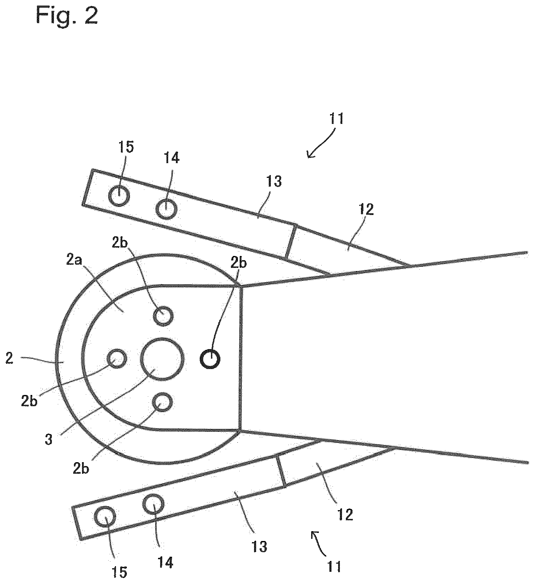

Fig. 1 is an explanatory view of one embodiment, which schematically shows the structure of a dental handpiece device 100. As can be seen from Fig. 1, the dental handpiece device 100 has the structure including a dental handpiece main body 1, and a dental handpiece imaging device 10. Fig. 2 is an enlarged view around a head 2 of the dental handpiece main body 1, which is a plan view seen from a side where a tool 3 is mounted.

In this embodiment, a known dental handpiece main body can be used as the dental handpiece main body 1. That is, the head 2 is included, and a tool mounted part 2a is provided for one face side of the head 2, on which the tool 3 such as a dental drill is mounted. Blowing air into a passage provided for the inside of the dental handpiece main body 1 gives the tool 3 a turning force.

As shown in Fig. 2, in this embodiment, a face of the head 2 where the tool mounted part 2a is formed includes four cooling water ejecting outlets 2b. The cooling water ejecting outlets 2b communicate with a passage provided for the inside of the dental handpiece main body 1. Passing water through the passage leads to ejection of cooling water from the cooling water ejecting outlets 2b.

While the embodiment of the dental handpiece main body 1 is given here as one example, the present invention is not limited to this, and a known dental handpiece main body can be applied as described above.

As can be seen from Fig. 1, the dental handpiece imaging device 10 includes image obtaining devices 11, a processing means 21, and a display means 31.

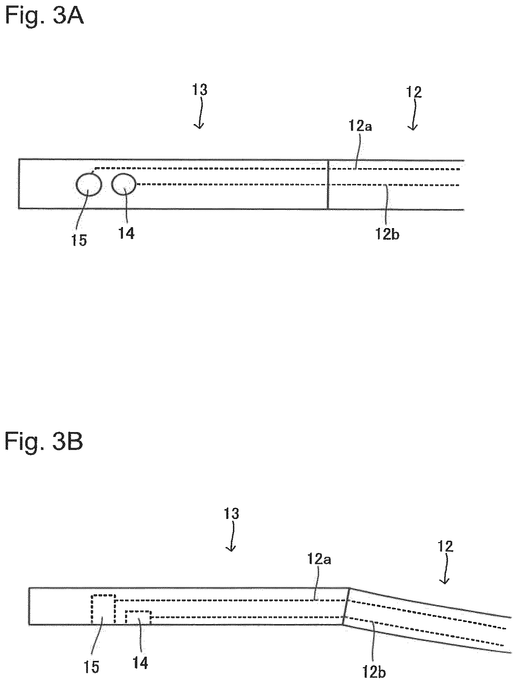

The image obtaining devices 11 are devices that obtain images of a scene of drilling a tooth, convert the images to electric signals, and transsmit the signals to the processing means 21. As can be seen from Fig. 2, a plurality of the image obtaining devices 11 are provided in this embodiment. Here, two image obtaining devices 11 are shown. However, the image obtaining device(s) 11 may be further provided at (an)other position(s). Fig. 3A and 3B are explanatory views of a tip portion of one image obtaining device 11. Fig. 3A and 3B show members included inside using dotted lines. Fig. 3A is a view seen from the same viewpoint as Fig. 2, and Fig. 3B is a view seen from the same viewpoint as Fig. 1.

The image obtaining device 11 has the structure including an electric conducting means 12, a light entry and outgo means 13, and retention members 16.

The electric conducting means 12 is a tubular member having the structure including conducting wires thereinside which electrically connect the light entry and outgo means 13 and the processing means 21, which will be described later. The electric conducting means 12 includes what is called conducting wires in a tubular inside thereof. At least two conducting wires are arranged in the electric conducting means 12. One of the conducting wires is a conducting wire 12a that connects a camera module 15 and the processing means 21, and the other is a conducting wire 12b that connects a light source 14 and the processing means 21.

The light entry and outgo means 13 is a tubular member that is arranged at one end of the electric conducting means 12, as can be seen from Figs. 1 to 3B. The light entry and outgo means 13 has the light source 14 including a light source light output part, and the camera module 15 including a light entry part having an inlet for incident light from an oral cavity, in a tubular inside thereof.

The light source 14 is a means of making illumination light outgo in response to the electric supply from a power source 22 of the processing unit 21 via the conducting wire 12b. Whereby the inside of an oral cavity is illuminated. Therefore, white light is preferable. A specific form of the light source 14 is not limited, and examples thereof include a LED in view of power consumption and a lifetime.

The camera module 15 is a means of properly letting light from the inside of an oral cavity which is to be photographed as an image enter, and converting this to an electric signal. The converted signal is transmitted to the processing means 21 by means of the conducting wire 12a. A specific form of the camera module 15 is not limited, and examples thereof include a device including a lens, and a photoelectric conversion sensor such as a CCD and a CMOS.

The retention members 16 are members provided so as to protrude from the electric conducting means 12 as can be seen from Fig. 1. The retention members 16 are means that make it possible to attach the electric conducting means 12, and the light entry and outgo means 13 to the dental handpiece main body 1, to retain them. A specific form of the retention members 16 is not limited as long as making such retention possible. Examples thereof include one having a structure so that the dental handpiece main body 1 is sandwiched in the retention member(s) 16, and one having a structure so as to adsorb the dental handpiece main body 1.

Here, the retention members have only to be able to retain the light entry and outgo means to the dental handpiece main body, and may be provided for the light entry and outgo means.

The image obtaining devices 11 as described above are attached and retained to the dental handpiece main body 1, to be a dental handpiece. That is, as can be seen from Figs. 1 and 2, the retention members 16 are attached to the dental handpiece main body 1. At this time, a plurality of the camera modules 15 of the image obtaining devices 11 are arranged so as to be focused on the tip of the tool 3 in different directions.

Returning to Fig. 1, the other components of the dental handpiece imaging device 10 will be described.

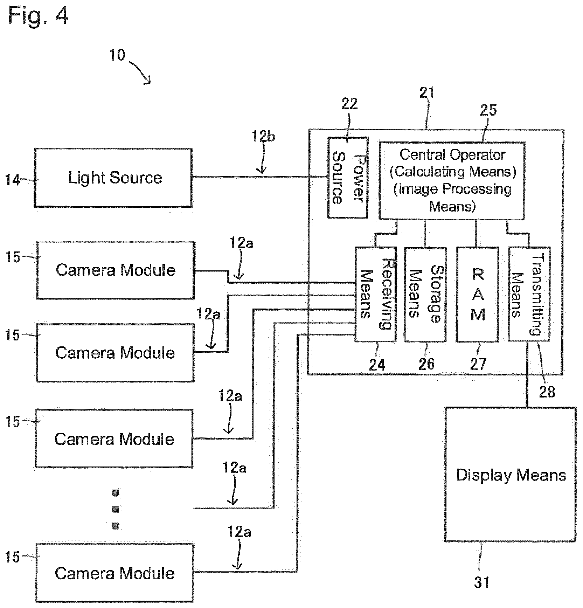

The processing means 21 has the power source 22 for the light sources 14 of the image obtaining devices 11, and is a means of processing image signals obtained from a plurality of the camera modules 15 of the image obtaining devices 11, and controlling a display. The processing means 21 has the structure including the power source 22, a receiving means 24, a central operator 25, a storage means 26, a RAM 27, and a transmitting means 28. Fig. 4 is an explanatory block diagram also showing the relationship between the processing means 21, the light sources 14, and the camera modules 15.

The power source 22 is a power source for the light sources 14. Therefore, as can be seen from Figs. 1 and 4, the power source 22 is connected to the light sources 14 via the conducting wires 12b of the electric conducting means 12.

The receiving means 24 is a member having a function of taking in electrical signals from the camera modules 15 which are based on the light from the inside of an oral cavity. The receiving means 24 is connected to a plurality of the camera modules 15. What is called an input port, an input connector, etc. are included therein. The manner of the connection is not limited, and may be either wired or wireless connection.

The central operator 25 is what is called a CPU, and functions as an image processing means, and other various calculating means. That is, the central operator 25 carries out various programs stored in the storage means 26 that functions as a storage medium, calculates and outputs results thereof, and controls the dental handpiece imaging device 10.

When functioning as an image processing means, the central operator 25 takes in an electrical signal from the receiving means 24, calculates based on programs, and as a result, generates an image signal. As one of the calculations, a calculation of determining a proper image among images from a plurality of the camera modules 15, to switch an image as needed as described later is included. Whereby, it is prevented that a portion drilled by the tool 3 is into an invisible state due to a blind spot. A specific manner thereof will be described later.

In addition, such an iconic effect can be given to the generated image signal as displaying the received signal as it is as an image, displaying images from a plurality of the camera modules 15 on a screen at the same time, partially enlarging an image, displaying part of an image so as to highlight the part, and measuring on a screen. Whereby, the convenience for a practitioner is improved. Known programs can be applied as programs for such image processing.

The central operator 25 is connected to other members provided for the processing means 21 such as the receiving means 24, the storage means 26, the RAM 27, and the transmitting means 28, and is configured so as to control them based on programs.

The storage means 26 is a member that functions as a storage medium where various programs and data that are the bases of calculations carried out in the central operator 25 are stored. The storage means 26 may further function as a storing means where data of images to be stored, and patients' data are stored.

The RAM 27 is a member that functions as a work area for calculations of the central operator 25, and a storage means for temporary data. The RAM 27 can be configured by a SRAM, a DRAM, flash memory, or the like, which is the same as a known RAM.

The transmitting means 28 is a member having a function of transmitting a signal of the obtained results which is to be transmitted to the display means 31. What is called an output port, an output connector, etc. are included therein. The manner of the transmission is not limited, and may be either wired or wireless transmission.

The display means 31 is connected to the transmitting means 28 of the processing means 21, and is a means of displaying an image based on information obtained from the transmitting means 28. Any monitor or the like can be used as the display means 31.

The dental handpiece imaging device 10, and the dental handpiece device 100 having the above described structures function as follows.

Blowing air into the dental handpiece main body 1 turns the mounted tool 3, and at the same time passing cooling water therethrough leads to ejection of the cooling water from the cooling water ejecting outlets 2b. Whereby, a tooth etc. can be drilled as usual while cooled.

In this embodiment, further, electricity is supplied from the power source 22 of the processing means 21, to light the light sources 14. This makes light outgo from the light sources 14, to illuminate the inside of an oral cavity. Illuminating the inside of an oral cavity as described above can lead to obtainment of an intraoral image. That is, reflected light from the illuminated inside of an oral cavity is captured by the camera modules 15, the processing means 21 generates images, and the images are displayed on the display means 31.

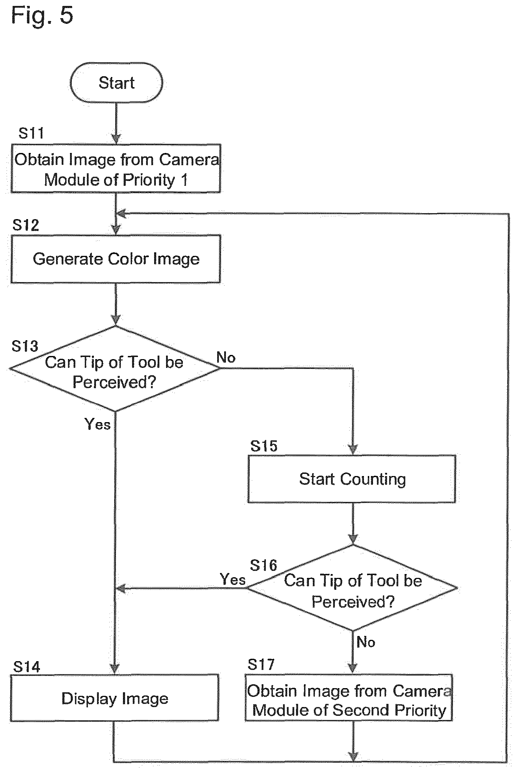

Here, the processing means 21 calculates, for example, as follows based on programs stored in the storage means 26 by means of the central operator that functions as an image processing means, to keep drilling condition by the tip of the tool 3 visually perceivable. Fig. 5 is a flowchart of one example of calculations of switching an image as needed. This flow includes steps S11 to S17.

First, as shown in the step S11, an image is obtained from a camera module 15 of priority 1, which is the first priority among a plurality of the camera modules 15, and a color image is generated by the processing means 21 as in the step S12. As can be found from the above, the priority order is decided in advance for a plurality of the camera modules 15.

After that, as shown in the step S13, it is determined whether the tip of the tool can be perceived with this camera module 15. If the tip of the tool can be perceived in this step, this means that drilling condition of a tooth can be visually perceived with this camera module. Thus, Yes is selected, the flow moves to the step S14, and the image is displayed. Returning to the step S12, the above steps are repeated.

In contrast, if the tip of the tool cannot be perceived with this camera module 15, No is selected in the step S13, and the flow moves to the step S15, to start counting. This step suspends for a predetermined time. This is because it is considered that the tip of the drilling tool is temporarily hard to be seen due to swarf, water drops attached by a spray of cooling water, etc. while a tooth is drilled. Thus, the camera module is not switched just after the drilling tool cannot be perceived, but a time limit counted by a counter is set, and only when the duration of a hidden state exceeds the set time, switching is carried out.

After a predetermined time has passed in the step S15, the flow moves to the step S16, and it is determined again whether the tip of the tool can be perceived with this camera module 15. If the tip of the tool can be perceived in this step, this means that the drilling condition of a tooth can be visually perceived with this camera module. Thus, Yes is selected, the flow moves to the step S14, and the image is displayed. Returning to the step S12, the above steps are repeated.

In contrast, if the tip of the tool cannot be perceived as well in this step, it can be determined that the drilling condition cannot be visually perceived with this camera module 15 in this position. Thus, the flow moves to the step S17, and an image from a camera module 15 of the second priority is obtained. After that, returning to the step S12, the above described steps are repeated.

As described above, according to the dental handpiece device 100 that is equipped with the dental handpiece imaging device 10, drilling can be performed while condition of a tooth etc. drilled by the tool 3 is visually perceived more certainly using the display means 31. Specifically, it can be prevented that a drilled portion is hidden by teeth etc., and cannot be seen while drilling, to improve the convenience for a practitioner.

The above description is one example of the control of the dental handpiece device 10 by the processing means 21. Modification can be added thereto as needed. For example, while an image from one camera module is displayed in this example, such a structure may be employed that images from a plurality of the camera modules can be displayed on a plurality of screens at the same time. Whereby, a drilled portion can be visually perceived from a plurality of viewpoints, which further improves convenience.

In addition to the above described structure of automatically switching a camera module, such a structure may be employed that a practitioner can manually switch a camera module. Whereby, an image can be selected according to the practitioner's preference, which is convenient. In this case, for example, a switch is provided somewhere for the dental handpiece device 100, and the processing means 21 can be configured so that an operation signal of this switch can switch and display one of a plurality of the camera modules. For example, whenever a practitioner pushes the switch, a camera module to be displayed is switched, which makes it possible to improve the convenience of a practitioner.

In the above embodiment, the handpiece main body 1 and the image obtaining device 11 are formed individually, and attachably and detachably. The present invention is not limited to this, and the handpiece main body 1 and the image obtaining device 11 may be made integrally.

### Reference Signs List

1 dental handpiece main body
2 head
2a tool mounted part
2b cooling water ejecting outlets
3 tool
10 dental handpiece imaging device
11 image obtaining device
12 electric conducting means
13 light entry and outgo means
14 light source
15 camera module
21 processing means
22 power source
24 receiving means
25 central operator
26 storage means
27 RAM
28 transmitting means
31 display means
100 dental handpiece device

## Claims

1. A dental handpiece device (100) comprising:
a dental handpiece main body (1); and
a dental handpiece imaging device (10) with which the dental handpiece main body (1) is equipped,
wherein the dental handpiece imaging device (10) comprises:
a plurality of camera modules (15);
a processing means (21); and
a display means (31);
wherein each of the plurality of camera modules (15) is adapted to take an image and to convert the image, which is taken, to an electric signal;
the processing means (21) is adapted to receive the electric signal from each of the camera modules (15), to calculate for generating an image based on the electric signal, and to switch a display of an image, which is taken by any of the plurality of camera modules, on the display means (31);
characterised therein that the processing means (21) is adapted to calculate for determining whether or not a portion drilled by a tool (3) that is mounted on the dental handpiece main body (1) can be visually perceived using one of the camera modules (15).

2. The dental handpiece device according to claim 1, wherein the processing means (21) is adapted to determine whether or not a tip of the tool (3) can be visually perceived, to determine whether the portion can be visually perceived.

3. The dental handpiece device according to claim 1 or 2, wherein
the processing means (21) is adapted to determine whether the portion can be visually perceived using one of the camera modules (15) which is set as a priority in advance, and
the processing means (21) is adapted to switch the camera module (15) of the priority to another one of the camera modules (15) which is set as a second priority, when determining that the portion cannot be visually perceived, to determine whether the portion can be visually perceived using the camera module (15) of the second priority.

4. The dental handpiece device according to claim 3, wherein
the processing means (21) is adapted to suspend for a predetermined time before switching the camera module (15) of the priority to the camera module (15) of the second priority.

5. The dental handpiece device according to claim 1, further comprising a switch which is adapted to switch an image, which is taken by one of the plurality of camera modules (15), by a signal from the switch.

## Patentansprüche

1. Zahnmedizinhandstückvorrichtung (100), umfassend:
einen Zahnmedizinhandstückhauptkörper (1); und
eine Zahnmedizinhandstückabbildungsvorrichtung (10), mit der der Zahnmedizinhandstückhauptkörper (1) ausgestattet ist,
wobei die Zahnmedizinhandstückabbildungsvorrichtung (10) umfasst:
eine Mehrzahl von Kameramodulen (15);
ein Verarbeitungsmittel (21); und
ein Anzeigemittel (31);
wobei jedes der Mehrzahl von Kameramodulen (15) dazu eingerichtet ist, ein Bild aufzunehmen und das aufgenommene Bild in ein elektrisches Signal umzuwandeln;
das Verarbeitungsmittel (21) dazu eingerichtet ist, das elektrische Signal von jedem der Kameramodule (15) zu empfangen, zu rechnen, um ein Bild auf der Grundlage des elektrischen Signals zu erzeugen, und eine Anzeige eines Bildes, das von irgendeinem der Mehrzahl von Kameramodulen aufgenommen wird, auf dem Anzeigemittel (31) zu schalten; **dadurch gekennzeichnet, dass** das Verarbeitungsmittel (21) dazu eingerichtet ist, zu rechnen, um zu bestimmen, ob ein Abschnitt, der von einem Werkzeug (3), das an dem Zahnmedizinhandstückhauptkörper (1) angebracht ist, gebohrt wird, unter Verwendung eines der Kameramodule (15) visuell wahrgenommen werden kann oder nicht.

2. Zahnmedizinhandstückvorrichtung nach Anspruch 1, wobei das Verarbeitungsmittel (21) dazu eingerichtet ist, zu bestimmen, ob eine Spitze des Werkzeugs (3) visuell wahrgenommen werden kann oder nicht, um zu bestimmen, ob der Abschnitt visuell wahrgenommen werden kann.

3. Zahnmedizinhandstückvorrichtung nach Anspruch 1 oder 2, wobei das Verarbeitungsmittel (21) dazu eingerichtet ist, zu bestimmen, ob der Abschnitt unter Verwendung eines der Kameramodule (15), das vorab als Priorität eingestellt ist, visuell wahrgenommen werden kann, und
das Verarbeitungsmittel (21) dazu eingerichtet ist, das Kameramodul (15) der Priorität auf ein anderes der Kameramodule (15), das als zweite Priorität eingestellt ist, umzuschalten, wenn festgestellt wird, dass der Abschnitt nicht visuell wahrgenommen werden kann, um zu bestimmen, ob der Abschnitt unter Verwendung des Kameramoduls (15) der zweiten Priorität visuell wahrgenommen werden kann.

4. Zahnmedizinhandstückvorrichtung nach Anspruch 3, wobei das Verarbeitungsmittel (21) dazu eingerichtet ist, für eine vorbestimmte Zeit vor dem Umschalten des Kameramoduls (15) der Priorität auf das Kameramodul (15) der zweiten Priorität zu pausieren.

5. Zahnmedizinhandstückvorrichtung nach Anspruch 1, weiter umfassend einen Schalter, der dazu eingerichtet ist, ein Bild, das von einem der Mehrzahl von Kameramodulen (15) aufgenommen wird, durch ein Signal des Schalters umzuschalten.

## Revendications

1. Dispositif de pièce à main dentaire (100) comprenant:
un corps principal de pièce à main dentaire (1); et
un dispositif d'imagerie de pièce à main dentaire (10) dont est équipé le corps principal de pièce à main dentaire (1),
dans lequel le dispositif d'imagerie de pièce à main dentaire (10) comprend:
une pluralité de modules de prise de vue (15);
un moyen de traitement (21); et
un moyen d'affichage (31);
dans lequel chacun de la pluralité de modules de prise de vue (15) est propre à prendre une image
et à convertir l'image, laquelle est prise, en un signal électrique;
le moyen de traitement (21) est propre à recevoir le signal électrique de chacun des modules de prise de vue (15),
pour effectuer un calcul dans le but de générer une image sur base du signal électrique, et pour commuter sur l'affichage d'une image, laquelle est prise par l'un quelconque de la pluralité des modules de prise de vue, sur le moyen d'affichage (31);
**caractérisé en ce que**
le moyen de traitement (21) est propre à effectuer un calcul pour déterminer si une partie forée par un outil (3) assemblé sur le corps principal de pièce à main dentaire (1) peut être ou non visuellement perçu en utilisant l'un des modules de prise de vue (15).

2. Dispositif de pièce à main dentaire selon la revendication 1, dans lequel le moyen de traitement (21) est propre à déterminer si un bout de l'outil (3) peut être ou non perçu visuellement, pour déterminer si la partie peut être visuellement perçue.

3. Dispositif de pièce à main dentaire selon la revendication 1 ou 2, dans lequel le moyen de traitement (21) est propre à déterminer si la partie peut être visuellement perçue en utilisant l'un des modules de prise de vue (15) établi prioritaire à l'avance, et
le moyen de traitement (21) est propre à commuter du module de prise de vue (15) prioritaire sur un autre des modules de prise de vue (15), lequel est établi secondairement prioritaire, lorsqu'il est déterminé que la partie ne peut être visuellement perçue, pour déterminer si la partie peut être visuellement perçue en utilisant le module de prise de vue (15) secondairement prioritaire.

4. Dispositif de pièce à main dentaire selon la revendication 3, dans lequel le moyen de traitement (21) est propre à suspendre, pendant une durée prédéterminée, la commutation du module de prise de vue (15) prioritaire à celui secondairement prioritaire.

5. Dispositif de pièce à main dentaire selon la revendication 1, comprenant en outre un commutateur propre à commuter d'une image, laquelle est prise par l'un de la pluralité des modules de prise de vue (15), sur un signal du commutateur.
